(19) Europäisches Patentamt
European Patent Office
Office européen des brevets

(11) Numéro de publication: 0 228 356
B1

# (12) FASCICULE DE BREVET EUROPÉEN

(45) Date de publication du fascicule du brevet: **02.08.89**

(51) Int. Cl.⁴: **C 07 D 405/12**, C 07 D 209/08

(21) Numéro de dépôt: **86870165.7**

(22) Date de dépôt: **06.11.86**

(54) **Nouveaux dérivés d'hydroxy-4 indole, leur procédé de préparation et leur utilisation.**

(30) Priorité: **12.11.85 FR 8516676**

(43) Date de publication de la demande: **08.07.87 Bulletin 87/28**

(45) Mention de la délivrance du brevet: **02.08.89 Bulletin 89/31**

(84) Etats contractants désignés: **AT BE CH DE ES FR GB GR IT LI LU NL SE**

(56) Documents cité:
**EP-A-0 005 828**
**EP-A-0 013 878**
**EP-A-0 025 111**

(73) Titulaire: **SANOFI S.A., 40, Avenue George V, F-75008 Paris (FR)**

(72) Inventeur: **Descamps, Marcel, Place des Carmes, 8/301, B-1300 Wavre (BE)**
Inventeur: **Verstraeten, Walter, Zwijvegemstraat, 10, B-2960 Mechelen (BE)**

(74) Mandataire: **Bressand, Georges, c/o CABINET LAVOIX 2 Place d'Estienne d'Orves, F-75441 Paris Cedex 09 (FR)**

**EP 0 228 356 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention se rapporte, d'une manière générale, à de nouveaux dérivés d'hydroxy-4 indole ainsi qu'à leur procédé de préparation.

Les dérivés d'hydroxy-4 indole de l'invention peuvent être représentés par la formule générale:

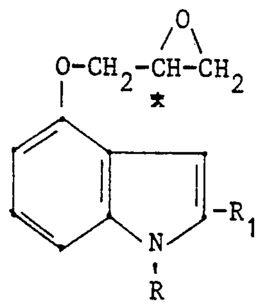

I

dans laquelle R représente un groupement protecteur labile et $R_1$ peut représenter:

- hydrogène,
- un radical alkyle, linéaire ou ramifié, ayant de 1 à 6 atomes de carbone,
- un radical cycloalkyle ayant de 3 à 6 atomes de carbone,
- un radical alkoxy inférieur,
- un radical hydroxyalkyle inférieur,
- un radical (alkoxy inférieur) alkyle inférieur,
- un radical phényle éventuellement substitué par un atome d'halogène ou par un radical alkyle inférieur ou alkoxy inférieur,
- un radical cyano,
- un radical de formule: 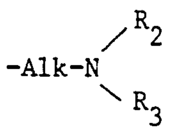 , 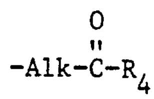 ou 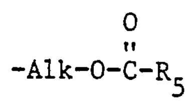

dans lequel:

$R_2$ et $R_3$, identiques ou différents, représentent chacun hydrogène ou un radical alkyle inferieur,

$R_4$ représente hydroxy, un radical alkyle inférieur ou alkoxy inférieur ou un radical 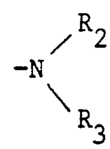

dans lequel $R_2$ et $R_3$ ont la même signification que précédemment,

$R_5$ représente un radical alkyle inférieur,

Alk représente une liaison simple ou un radical alkylène, linéaire ou ramifié, ayant de 1 à 4 atomes de carbone.

Dans le présent contexte, les termes repris ci-dessous comportent la signification suivante:

"groupement protecteur labile" désigne un groupement protecteur facilement éliminable tels que, à titre d'exemple, un groupement alkylcarbonyle tels que formyle, acétyle ou propionyle, un groupement arylcarbonyle tel que benzoyle, un groupement alkylsulfonyle tel que méthanesulfonyle ou un groupement arylsulfonyle tel que benzènesulfonyle ou p-toluènesulfonyle, le groupement arylsulfonyle et en particulier les groupements benzènesulfonyle et p-toluènesulfonyle constituant des groupement préférés;

"alkyle inférieur" désigne les restes d'hydrocarbures aliphatiques saturés linéaires ou ramifiés contenant jusqu'à 4 atomes de carbone tels que méthyle, éthyle, n-propyle, isopropyle, n-butyle, isobutyle ou tertiobutyle;

"alkoxy inférieur" désigne le groupement hydroxy substitué par un radical alkyle inférieur tel que défini précédemment.

Ainsi, tenant compte des valeurs données ci-dessus, $R_1$ peut représenter notamment hydrogène, un radical méthyle, éthyle, n-propyle, isopropyle, n-butyle, isobutyle, tertiobutyle, méthyl-1 propyle, n-pentyle, n-hexyle, cyclohexyle, méthoxy, éthoxy, hydroxyméthyle, hydroxy-1 éthyle, méthyloxyméthyle, phényle, chlorophényle, méthylphényle, méthoxyphényle, cyano, méthylamino, diméthylamino, méthylamino- ou éthylamino-éthyle, diméthylamino- ou diéthylamino-éthyle, carboxy, méthoxycarbonyle, éthoxycarbonyle, méthyl-2 méthoxycarbonyl-2 propyle, carbamoyle, N,N-diméthyl- ou N,N-diéthyl-carbamoyle, acétyle, propionyle, acétoxyméthyle ou pivaloyloxyméthyle. Les valeurs hydrogène et méthyle représentent cependant des valeurs préférées du radical $R_1$.

En raison du carbone asymétrique représenté par un astérisque, les composés de formule I peuvent se présenter sous la forme d'isomères lévogyre dextrogyre ou de mélanges de ces isomères.

L'invention se rapporte également aux dérivés d'hydroxy-4 indole sous la forme d'isomères lévogyre et dextrogyre séparés ou sous la forme de mélanges de ces isomères tel que sous la forme du mélange

racémique.

Les composés de l'invention se sont révélés particulièrement utiles comme produits intermédiaires notamment pour la préparation de dérivés d'(amino-3 hydroxy-2 propoxy)-4 indole de formule générale

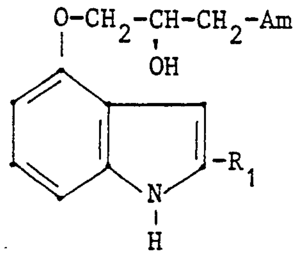

Ia

dans laquelle $R_1$ a la même signification que précédemment et Am représente un radical amino substitué ou non, ainsi que de leurs sels.

De tels produits sont décrits notamment dans les brevets U.S. Nos. 3 471 515 et 4 076 829 et français No. 8 067 M et dans la demande de brevet européen No. 25 727.

Ces composés présentent un intérêt incontestable en raison de leurs propriétés bloquantes des récepteurs β-adrénergiques les rendant utiles dans le domaine cardiovasculaire.

Un autre objet de l'invention se rapporte, en conséquence, aux dérivés d'hydroxy-4 indole de formule I en tant que produits industriels nouveaux utiles notamment comme intermédiaires par exemple pour la synthèse finale des dérivés (amino-3 hydroxy-2 propoxy)-4 indole de formule Ia.

On a décrit dans le brevet U.S. No. 3 471 515 et le brevet français No. 1 598 040 un procédé pour la préparation de dérivés (amino-3 hydroxy-2 propoxy)-4 indole par réaction de l'hydroxy-4 indole avec l'épichlorhydrine ou l'épibromhydrine sous forme racémique ou sous forme lévogyre, en milieu alcalin et en absence d'oxygène puis condensation des composés obtenus avec une amine.

Dans le cadre de l'élaboration de la présente invention, on a tenté de préparer des dérivés (amino-3 hydroxy-2 propoxy)-4 indole de formule Ia selon la méthode décrite précédemment tel qu'indiqué dans l'art antérieur c'est-à-dire sans isolation des composés formés après réaction de l'hydroxy-4 indole avec l'épichlorhydrine.

Cette méthode cependant n'a fourni que de faibles rendements en dérivé d'(amino-3 hydroxy-2 propoxy)-4 indole en question.

Par exemple, la condensation de l'hydroxy-4 indole avec la diméthyltryptamine n'a pas permis d'obtenir plus de 20 à 25 % de {[(1H-indolyl-3)-2 dimethyl-1,1 ethylamino]-3 hydroxy-2 propoxy}-4 indole de formule Ia.

On sait d'autre part que par réaction de l'hydroxy-4 indole et de l'épichlorhydrine, on obtient un mélange de composés constitués notamment d'oxyranylméthoxy-4 indole et, en plus faible proportion, de (chloro-3 hydroxy-2 propoxy)-4 indole.

En conséquence, on a également préparé le {[(1H-indolyl-3)-2 diméthyl-1,1 éthylamino]-3 hydroxy-2 propoxy}-4 indole par condensation de la diméthyltryptamine avec l'oxyranylméthoxy-4 indole isolé et purifié de son milieu de synthèse. [Helv. Chimica Acta, Vol. 54, p. 2411 (1971)].

Bien que les rendements en dérivé d'(hydroxy-2 propoxy)-4 indole produit de cette manière soient supérieurs à ceux obtenus sans purification intermédiaire de l'oxyranylméthoxy-4 indole, cette mise en oeuvre présente néanmoins plusieurs inconvénients tels qu'ils rendent le procédé difficilement exploitable sur grande échelle.

En effet, l'oxyranylméthoxy-4 indole étant un composé de point de fusion mal défini (entre 50 et 60°C) ne peut pas être purifié par cristallisation mais nécessite l'utilisation d'une méthode beaucoup plus difficilement extrapolable sur le plan industriel telle qu'une chromatographie sur colonne.

Au surplus, l'oxyranylméthoxy-4 indole est un produit très photosensible ce qui posera des problèmes pour sa conservation.

En conséquence, la crédibilité sur le plan industriel d'un procédé tel que celui décrit précédemment apparaît fortement compromise au vu des résultats et des inconvénients rencontrés.

On a maintenant trouvé, selon l'invention, que les dérivés d'hydroxy-4 indole de formule I ci-dessus constituent des composés particulièrement intéressants puisque, par leur intermédiaire, les dérivés d'(amino-3 hydroxy-2 propoxy)-4 indole de formule Ia peuvent être obtenus avec des rendements globaux supérieurs à ceux obtenus selon la technique antérieure tout en evitant les inconvénients de cette dernière.

En effet, les composés de l'invention présentent un avantage incontestable par rapport à l'oxyranylméthoxy-4 indole: ils peuvent être utilisés à l'état brut pour la production des composés de formule Ia avec rendements importants.

Par conséquent, leurs isolation et purification s'avèrent inutiles au contraire de l'oxyranylméthoxy-4 indole qui nécessite ces phases supplémentaires de procédé pour l'obtention de rendements significatifs en composé de formule Ia.

En fait, les composés de l'invention se sont révélés beaucoup plus stables que l'oxyranylméthoxy-4 indole et de point de fusion plus net que celui de ce composé. Par conséquent, les composés de formule Ia peuvent être isolés, si nécissaire, selon des techniques applicables industriellement par exemple par cristallisation et leur grande stabilité à la lumière pourra être mise à profit en cas de nécessité de conservation.

Parmi les dérivés d'(amino-3 hydroxy-2 propoxy)-4 indole de formule Ia ci-dessus, l'(isopropylamino-3 hydroxy-2 propoxy)-4 indole ou pindolol et l'(isopropylamino-3 hydroxy-2 propoxy)-4 méthyl-2 indole ou

mépindolol constituent des composés pharmaceutiques particulièrement intéressants de même qu'un autre dérivé analogue à savoir le (tertiobutylamino-3 benzoyloxy-2 propoxy)-4 méthyl-2 indole ou bopindolol.

En conséquence, les composés de formule I capables de donner accès à ces composés pharmaceutiques constitueront des dérivés préférés selon l'invention, c'est-à-dire les composés de formule I dans laquelle R a la signification donnée par exemple arylsulfonyle, de préférence benzènesulfonyle ou p-toluènesulfonyle et $R_1$ représente hydrogène ou méthyle.

Ainsi les composés de formule Ia ainsi que leurs sels peuvent être obtenus, selon l'invention en traitant au reflux un composé de formule I ci-dessus avec une amine de formule générale:

H-Am II

dans laquelle Am représente un groupement amino substitué ou non par exemple isopropylamino, tertiobutylamino ou (1H-indolyl-3)-2 diméthyl-1,1 éthylamino et ce, dans un solvant approprié tel qu'un alcool inférieur par exemple l'éthanol, le n-propanol ou l'isopropanol ou tel qu'un excès d'amine de formule II de manière à former un dérivé d'(hydroxy-2 propoxy)-4 indole de formule générale:

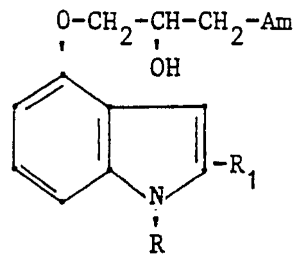

III

dans laquelle R, $R_1$ et Am ont la même signification que précédemment, dérivé que l'on déprotège, de manière classique, par chauffage au reflux dans un solvant approprié tel qu'un alcool inférieur par exemple l'éthanol, le n-propanol ou l'isopropanol et en présence d'un hydroxyde de métal alcalin tel que l'hydroxyde de sodium, ce qui fournit le composé désiré de formule Ia que l'on peut faire réagir, si on le désire, avec un acide pour former un sel.

Généralement le composé de formule III est isolé de son milieu de préparation sous forme d'un sel d'addition par exemple sous forme de chlorhydrate.

Cette méthode permet l'obtention de dérivés d'(amino-3 hydroxy-2 propoxy)-4 indole de formule Ia avec de bons rendements ceux-ci pouvant s'élever, dans certains cas, à au moins 75 % à partir du composé de formule I.

Les dérivés d'hydroxy-4 indole de l'invention, quant à eux, peuvent être obtenus en faisant réagir un dérivé d'indole N-protégé de formule générale:

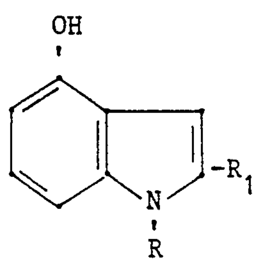

IV

dans laquelle R et $R_1$ ont la même signification que précédemment avec l'épichlorhydrine ou l'épibromhydrine en présence d'un agent de métallation et éventuellement en présence d'un catalyseur de transfert de phase.

Comme agent de métallation on peut envisager tout composé basique capable de salifier l'hydroxy-4 du dérivé de formule IV par exemple un hydrure de métal alcalin tel que l'hydrure de sodium ou de potassium, un hydroxyde de métal alcalin tel que l'hydroxyde de sodium ou de potassium, un carbonate de métal alcalin par exemple le carbonate de sodium ou de potassium ou un alcoolate de métal alcalin tel que le méthanolate de sodium.

La réaction a lieu à la température de reflux et dans un milieu approprié monophasique généralement un hydrocarbure aromatique tel que le toluène, un alcool inférieur tel que le méthanol ou encore dans un milieu diphasique tel qu'un mélange eau/chlorure de méthylène.

En outre, l'épichlorhydrine ou l'épibromhydrine utilisée sera sous la forme d'isomère dextrogyre ou lévogyre ou sous la forme de mélange de ces isomères par exemple sous forme de racémique de manière à produire les composés de formule I respectivement sous la forme d'isomère dextrogyre ou lévogyre ou sous la forme de mélange de ces isomères.

Quant au catalyseur de transfert de phase, celui-ci sera par exemple un sel de tétrabutylammonium tel que le chlorure ou l'hydro de préférence un dérivé tris(dioxa-alkyl)amine tel que la tris(dioxa-3,6 heptyl)amine ou TDA-1.

On a en effet remarqué que les dérivés d'hydroxy-4 indole de l'invention peuvent être obtenus avec des rendements pratiquement quantitatifs à partir du dérivé d'indole N-protégé de formule IV, en particulier à partir du benzènesulfonyl-1 ou p-toluènesulfonyl-1 oxyranylméthoxy-4 indole lorsque la réaction a lieu en présence d'un tris(dioxa-alkyl)amine de préférence la tris(dioxa-3,6 heptyl)amine comme catalyseur de transfert de phase.

Mis en oeuvre à l'état brut, le dérivé de formule I ainsi obtenu peut fournir d'importants rendements en composé de formule Ia, ceux-ci pouvant dépasser, dans certains cas, 70 % à partir de l'hydroxy-4 indole N-protégé de formule IV.

En conséquence, un autre objet de l'invention se rapporte à un procédé de préparation des dérivés d'(amino-3 hydroxy-2 propoxy)-4 indole de formule Ia ci-dessus ainsi que de leurs sels, procédé selon lequel:

- on fait réagir au reflux et dans un milieu approprié tel que décrit précédemment, un dérivé d'indole N-protégé de formule IV avec l'épichlorhydrine ou l'épibrohydrine en présence d'un agent de métallation tel que décrit précédemment et d'un dérivé tris(dioxa-alkyl)amine de préférence la tris(dioxa-3,6 heptyl)amine comme catalyseur de transfert de phase pour obtenir un composé de formule I à l'état brut,
- on traite au reflux le composé de formule I brut ainsi obtenu avec une amine de formule II et ce, dans un solvant approprié tel que décrit précédemment de manière à former un dérivé d'(hydroxy-2 propoxy)-4 indole de formule III,
- on déprotège le composé de formule III par chauffage au reflux dans un solvant approprié, comme décrit précédemment et en présence d'un hydroxyde de métal alcalin pour obtenir le composé désiré de formule Ia, que l'on peut, si on le désire, faire réagir avec un acide pour former un sel de ce composé.

Les dérivés d'indole de formule IV sont des produits nouveaux à l'exception du p-toluènesulfonyl-1 hydroxy-4 indole qui est décrit dans la demande de brevet japonais No. 142968/82 (C.A. 98: 7190 j).

Ces dérivés d'indole de formule IV peuvent être obtenus en traitant au reflux un dérivé de tétrahydroindole de formule générale:

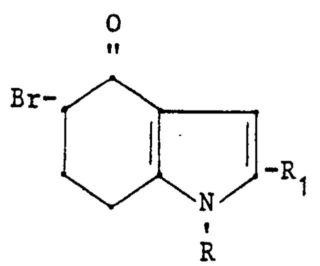

V

dans laquelle R et $R_1$ ont la même signification que précédemment, au moyen d'une amine tertiaire telle que la triéthylamine et dans un solvant approprié tel qu'un solvamt non polaire par exemple le toluène.

Les composés de formule V, quant à eux, peuvent être préparés selon la méthode décrite dans le brevet U.S. No. 3 654 303.

Les Exemples non limitatifs suivants illustrent l'invention:

**Exemple 1**

Préparation du p-toluènesulfonyl-1 oxyranylméthoxy-4 indole

Dans un ballon de 500 ml muni d'un réfrigérant et d'une arrivée d'azote, on introduit 32,6 g (0,113 mole) de p-toluènesulfonyl-1 hydroxy-4 indole, 230 ml de méthanol, 20 g (0,144 mole) de carbonate de potassium et 30,7 g (0,331 mole) d'épichlorhydrine.

On porte le mélange au reflux durant 4 h et sous atmosphère d'azote puis on amène à sec sous vide. On ajoute environ 100 ml de chlorure de méthylène et environ 10 ml d'eau puis on extrait deux fois avec du chlorure de méthylène. On lave la phase organique à l'eau puis trois fois avec de l'hydroxyde de sodium à 5 % et enfin, trois fois avec de l'eau. On sèche, décolore et filtre. On amène à sec sous vide, on ajoute un peu d'éther éthylique et on place au réfrigérateur pour provoquer la précipitation du composé désiré puis on filtre.

De cette manière, on obtient 17,5 g de p-toluènesulfonyl-1 oxyranyl-méthoxy-4 indole, ce qui représente un rendement de 45,1 %.

P.F.: 116 - 119°C.

**Exemple 2**

Préparation du p-toluènesulfonyl-1 oxyranylméthoxy-4 indole

Dans un ballon de 250 ml, on introduit 2,87 g (0,01 mole) de p-toluènesulfonyl-1 hydroxy-4 indole, 0,34 g d'hydrogénosulfate de tétrabutylammonium et 45 ml de chlorure de méthylène. On agite durant 5 min. puis on ajoute 10 ml d'une solution aqueuse à 50 % d'hydroxyde de sodium. On agite à nouveau durant 5 min. et on introduit 1,18 ml (0,015 mole) d'épichlorhydrine.

Lors de l'addition de l'hydroxyde de sodium un précipité apparaît. On agite durant 3 heures puis on ajoute 20 ml d'eau pour provoquer la solubilisation du précipité. On agite à nouveau durant 3 heures et on chauffe au reflux pendant environ 6 heures. On ajoute 1 ml d'épichlorhydrine et on chauffe au reflux durant 3 heures. On décante, lave la phase organique à l'eau et sèche. On filtre et amène à sec sous vide jusqu'à l'obtention d'un résidu pâteux. On ajoute de l'éther éthylique et on filtre. On amène le filtrat à sec sous vide, on ajoute un peu d'éther éthylique et on maintient au réfrigérateur pour provoquer la précipitation du composé désiré puis on filtre.

De cette manière, on obtient 1 g de p-toluènesulfonyl-1 oxyranylméthoxy-4 indole, ce qui représente un rendement de 29 %.

**Exemple 3**

Préparation du benzènesulfonyl-1 oxyranylméthoxy-4 indole

Dans un ballon de 100 ml muni d'un thermomètre, d'un réfrigérant et d'un agitateur, on introduit 2,5 g (0,09 mole) de benzènesulfonyl-1 hydroxy-4 indole, 20 ml de méthanol, 1,53 g de carbonate de potassium et 2 ml d'épichlorhydrine. On agite durant 5 minutes et on chauffe à 90°C (bain-marie) pendant 30 minutes. On coule la solution dans de l'eau et on extrait deux fois avec du chlorure de méthylène. On lave la phase organique avec une solution aqueuse diluée d'hydroxyde de potassium puis deux fois avec de l'eau. On sèche, on évapore à sec et on cristallise le composé désiré dans l'isopropanol.

De cette manière, on obtient le benzènesulfonyl-1 oxyranylméthoxy-4 indole avec un rendement de 33 %.

P.F.: 96°C.

**Exemple 4**

Préparation de l'(isopropylamino-3 hydroxy-2 propoxy)-4 indole ou pindolol

a) p-Toluènesulfonyl-1 oxyranylméthoxy-4 indole

On porte au reflux 350 g (1,232 mole) de p-toluènesulfonyl-1 hydroxy-4 indole dans 11 de toluène de manière à éliminer l'eau. Après refroidissement, on ajoute 54 g (1,35 mole) d'hydrure de sodium à 55 %. On chauffe le mélange au reflux et on amène à sec sous vide. On introduit alors 1,302 g (14 moles) d'épichlorhydrine et 19,7 ml (0,06 mole) de tris(dioxa-3,6 heptyl) amine. On porte à 100°C pendant 4 heures sous courant d'azote, on amène à sec sous vide puis on ajoute environ 1000 ml de chlorure de méthylène et environ 100 ml d'eau. On agite et sépare le mélange puis on lave la phase organique deux fois avec de l'eau. On sèche sur sulfate de sodium anhydre, on décolore et on filtre. Après cette opération, on amène le filtrat à sec sous vide.

De cette manière, on obtient environ 420 g de p-toluènesulfonyl-1 oxyranylméthoxy-4 indole brut ce qui représente un rendement de 100 %. Pureté: 95,79 %

P.F.: 109 - 112°C.

En utilisant le même procédé que celui décrit ci-dessus, on a préparé le benzènesulfonyl-1 oxyranylméthoxy-4 indole avec un rendement d'environ 100 %.

Pureté: 94,6 %

b) Chlorhydrate de p-toluènesulfonyl-1 (isopropylamino-3 hydroxy-2 propoxy)-4 indole

Dans un flacon de Parr, on introduit 17 g (0,05 mole) de p-toluène-sulfonyl-1 oxyranylméthoxy-4 indole tel qu'obtenu précédemment et 250 ml d'isopropylamine puis on chauffe à 100°C. Après 48 heures, la réaction est complète. On évapore à sec sous vide et on précipite le chlorhydrate par addition d'acétate d'éthyle chlorhydrique. On agite durant une heure, on filtre et on lave avec de l'acétate d'éthyle.

De cette manière, on obtient 12 g de chlorhydrate de p-toluènesulfonyl-1 (isopropylamino-3 hydroxy-2 propoxy)-4 indole après 2 recristallisations dans l'éthanol.

Rendement: 54,6 %

P.F.: 223 - 224°C

Spectres RMN, IR: corrects.

Analyse:

| | C % | H % | N % |
|---|---|---|---|
| Calculé | 57,46 | 6,20 | 6,38 |
| Trouvé | 57,10 | 6,36 | 6,12 |

c) (Isopropylamino-3 hydroxy-2 propoxy)-4 indole

Dans un ballon de 250 ml muni d'un réfrigérant, on introduit 10 g (0,027 mole) de chlorhydrate de p-toluènesulfonyl-1 (isopropylamino-3 hydroxy-2 propoxy)-4 indole, 60 ml d'éthanol et 4 g (0,1 mole) d'hydroxyde de sodium dans 22 ml d'eau. On chauffe durant 4 heures au reflux sous azote et on laisse reposer pendant 12 h. Le produit désiré cristallise. On le filtre et on le recristallise dans l'isopropanol.

De cette manière, on obtient 4,5 g d'(isopropylamino-3 hydroxy-2 propoxy)-4 indole.

Rendement: 69,6 %

P.F.: 162°C

Pureté: 100 %

Analyse:

| | C % | H % | N % |
|---|---|---|---|
| Calculé | 67,71 | 8,12 | 11,28 |
| Trouvé | 67,93 | 8,21 | 11,47 |

**Exemple 5**

Préparation du chlorhydrate de {[(1H-indolyl-3)-2 diméthyl-1,1 éthylamino]-3 hydroxy-2 propoxy}-4 indole

a) Chlorhydrate de p-toluènesulfonyl-1 {[(1H-indolyl-3)-2 diméthyl-1,1 éthylamino]-3 hydroxy-2 propoxy}4 indole.

Dans un ballon de 31, on introduit 172 g (0,5 mole) de p-toluène-sulfonyl-1 oxyranylméthoxy-4 indole tel qu'obtenu à l'Exemple 4a, 94,13 g (2 moles) de diméthyltryptamine et 21 d'isopropanol. Sous atmosphère d'azote, on chauffe au reflux durant 12 heures puis on porte à sec. On reprend le résidu dans 41 d'acétate d'éthyle et on ajoute une solution de chlorure d'hydrogène gazeux dans l'acétate d'éthyle. On agite durant 2 h, filtre et lave le précipité avec de l'acétate d'éthyle.

De cette manière, on obtient 220 g de chlorhydrate de p-toluènesulfonyl-1 {[(1H-indolyl-3)-2 diméthyl-1,1 éthylamino]-3 hydroxy-2 propoxy}4 indole.

Rendement: 77,5 %

Pureté: 94,54 %

Spectres RMN, IR: conformes

Analyse:

| | C % | H % | N % | S % | Cl % |
|---|---|---|---|---|---|
| Calculé | 63,42 | 6,03 | 7,40 | 5,64 | 6,24 |
| Trouvé | 63,77 | 5,94 | 7,32 | 5,67 | 6,34 |

b) Chlorhydrate de {1(1H-indolyl-3)-2 diméthyl-1,1 éthylamino]-3 hydroxy-2 propoxy}4 indole

Dans un ballon de 31, on introduit 200 g (0,352 mole) de chlorhydrate de p-toluènesulfonyl-1 {[(1H-indolyl-3)-2 diméthyl-1,1 éthylamino]-3 hydroxy-2 propoxy}4 indole, 900 ml d'éthanol et 61 g (0,655 mole) d'hydroxyde de sodium dans 325 ml d'eau. On chauffe durant 4 h au reflux sous atmosphère d'azote puis on coule le mélange dans 141 d'eau. On agite durant 1 h, on lave et on sèche, ce qui fournit 130 g de {[(1H-indolyl-3)-2 diméthyl-1,1 éthylamino]-3 hydroxy-2 propoxy}4 indole sous forme de base.

Rendement: 100 %

Pureté: 95,77 %

On dissout la base obtenue dans l'isopropanol et on filtre l'insoluble. On ajoute à la solution obtenue une solution d'isopropanol chlorhydrique, ce qui provoque, par agitation, la précipitation du chlorhydrate après quelques heures. On filtre et on lave avec de l'isopropanol.

On obtient de cette manière le chlorhydrate de {[(1H-indolyl-3)-2 diméthyl-1,1 éthylamino]-3 hydroxy-2 propoxy}4 indole.

P.F.: 95 - 113°C

**Revendications** pour les Etats contractants: BE, CH, DE, FR ,GB, GR, IT, LI, LU, NL, SE

1. Dérivés d'hydroxy-4 indole de formule générale:

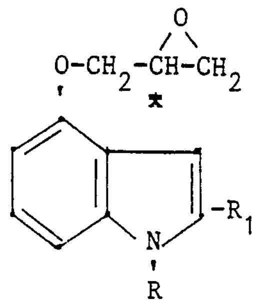

dans laquelle R représente un groupement protecteur labile et $R_1$ peut représenter:

- hydrogène,
- un radical alkyle, linéaire ou ramifié en $C_1$-$C_6$,
- un radical cycloalkyle en $C_3$-$C_6$,
- un radical alkoxy en $C_1$-$C_4$,
- un radical hydroxyalkyle en $C_1$-$C_4$,
- un radical (alkoxy en $C_1$-$C_4$) alkyle en $C_1$-$C_4$,
- un radical phényle éventuellement substitué par un atome d'halogène ou par un radical alkyle en $C_1$-$C_4$ ou alkoxy en $C_1$-$C_4$,
- un radical cyano,
- un radical de formule:

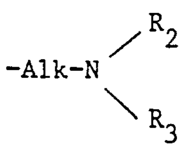 , 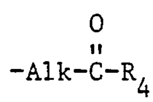 ou 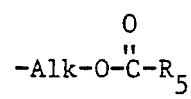

dans lequel: $R_2$ et $R_3$, identiques ou différents, représentent chacun hydrogène ou un radical alkyle en $C_1$-$C_4$,

$R_4$ représente hydroxy, un radical alkyle en $C_1$-$C_4$ ou alkoxy $C_1$-$C_4$ ou un radical FIGUR

dans lequel $R_2$ et $R_3$ ont la même signification que précédemment,
$R_5$ représente un radical alkyle en $C_1$-$C_4$,
Alk représente une liaison simple ou un radical alkylène, linéaire ou ramifié, ayant de 1 à 4 atomes de carbone.

2. Dérivés d'hydroxy-4 indole selon la Revendication 1 dans laquelle R représente un groupement alkylcarbonyle, arylcarbonyle, alkylsulfonyle ou arylsulfonyle.

3. Dérivés d'hydroxy-4 indole selon la Revendication 1 dans laquelle R représente un radical formyle, acétyle, propionyle, benzoyle, méthanesulfonyle, benzènesulfonyle ou p-toluènesulfonyle.

4. Dérivés d'hydroxy-4 indole selon la Revendication 1 dans laquelle $R_1$ représente hydrogène, un radical méthyle, éthyle, n-propyle, isopropyle, n-butyle, isobutyle, tertiobutyle, méthyl-1 propyle, n-pentyle, n-hexyle, cyclohexyle, méthoxy, éthoxy, hydroxyméthyle, hydroxy-1 éthyle, méthyloxyméthyle, phényle, chlorophényle, méthylphényle, méthoxyphényle, cyano, méthylamino, diméthylamino, méthylamino- ou éthylamino-éthyle, diméthylamino- ou diéthylamino-éthyle, carboxy, méthoxycarbonyle, éthoxycarbonyle, méthyl-2 méthoxycarbonyl-2 propyle, carbamoyle, N,N-diméthyl- ou N,N-diéthylcarbamoyle, acétyle, propionyle, acétoxyméthyle ou pivaloyloxyméthyle.

5. Dérivés d'hydroxy-4 indole selon la Revendication 1 dans laquelle $R_1$ représente hydrogène ou méthyle et R représente benzènesulfonyle ou p-toluènesulfonyle.

6. Benzènesulfonyl-1 oxyranylméthoxy-4 indole.

7. p-Toluènesulfonyl-1 oxyranylméthoxy-4 indole.

8. Procédé de préparation de dérivés d'hydroxy-4 indole selon une des Revendications 1 à 7 caractérisé en ce que l'on fait réagir, dans un milieu approprié et à la température de reflux, un dérivé d'indole N-protégé de formule générale:

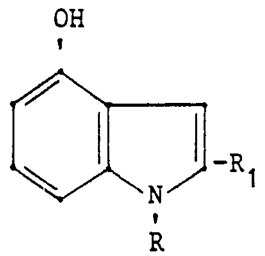

dans laquelle R et $R_1$ ont la même signification que dans les Revendications 1 à 5 avec l'épichlorhydrine ou l'épibromhydrine en présence d'un agent de métallation et éventuellement en présence d'un catalyseur de transfert de phase.

9. Procédé de préparation de dérivés d'(amino-3 hydroxy-2 propoxy)-4 indole de formule générale:

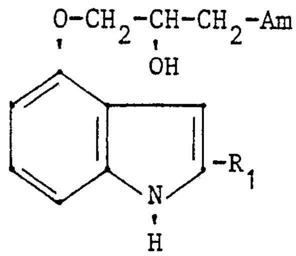

ainsi que de leurs sels, dans laquelle $R_1$ a la même signification que dans l'une des Revendications 1 à 5 et Am représente un groupement amino substitué ou non caractérisé en ce que l'on traite au reflux un dérivé d'hydroxy-4 indole selon une des Revendications 1 à 5, avec une amine de formule générale:

H-Am

dans laquelle Am a la même signification que précédemment et ce, dans un alcool inférieur ou un excès d'amine ci-dessus, de manière à former un dérivé d'(hydroxy-2 propoxy)-4 indole de formule générale:

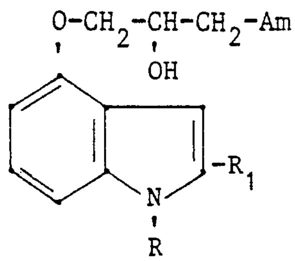

dans laquelle R et $R_1$ ont la même signification que dans les Revendications 1 à 5 et Am a la même signification que précédemment, dérivé que l'on déprotège par chauffage au reflux dans un alcool inférieur et en présence d'un hydroxyde de métal alcalin pour obtenir le dérivé désiré d'(amino-3 hydroxy-2 propoxy)-4 indole que l'on peut faire réagir, si on le désire, avec un acide pour former un sel de ce composé.

10. Procédé de préparation de dérivés d'(amino-3 hydroxy-2 propoxy)-4 indole de formule générale:

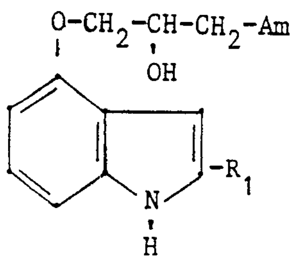

ainsi que de leurs sels, dans laquelle $R_1$ a la même signification que dans l'une des Revendications 1 à 5 et Am représente un groupement amino substitué ou non caractérisé en ce que:

- l'on fait réagir, dans un milieu approprié et à la température de reflux, un dérivé d'indole N-protégé de formule générale:

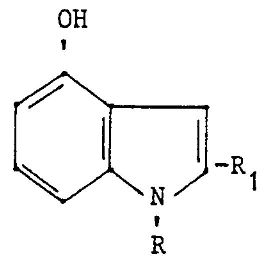

dans laquelle R et $R_1$ ont la même signification que dans les Revendications 1 à 5, avec l'épichlorhydrine ou l'épibromhydrine en présence d'un agent de métallation et d'un tris(dioxa-alkyl)amine comme catalyseur de transfert de phase pour obtenir un dérivé d'hydroxy-4 indole sous forme brute selon une des Revendications 1 à 7,

- l'on traite le dérivé d'hydroxy-4 indole brut ainsi obtenu avec une amine de formule générale:

H-Am

dans laquelle Am a la même signification que précédemment et ce, dans un alcool inférieur ou un excès d'amine ci-dessus, de manière à former un dérivé d'(hydroxy-2 propoxy)-4 indole de formule générale:

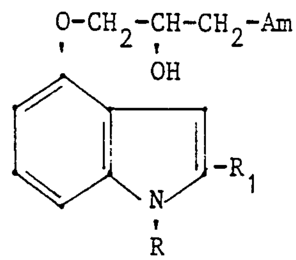

dans laquelle R et $R_1$ ont la même signification que dans les Revendications 1 à 5 et Am a la même signification que précédemment,

- l'on déprotège le dérivé d'(hydroxy-2 propoxy)-4 indole ainsi obtenu, par chauffage au reflux dans un alcool inférieur et en présence d'un hydroxyde de métal alcalin pour obtenir le dérivé désiré d'(amino-3 hydroxy-2 propoxy)-4 indole que l'on peut faire réagir, si on le désire, avec un acide pour former un sel.

11. Procédé selon la Revendication 8 ou 10 caractérisé en ce que l'agent de métallation est un hydrure de métal alcalin, un hydroxyde de métal alcalin, un carbonate de métal alcalin ou un alcoolate de métal alcalin.

12. Procédé selon la Revendication 8 ou 10 caractérisé en ce que le catalyseur de transfert de phase est la tris(dioxa-3,6 heptyl)amine.

13. Procédé selon la Revendication 8 ou 10 caractérisé en ce que le milieu est monophasique ou diphasique.

14. Procédé selon la Revendication 8 ou 10 caractérisé en ce que Am représente un groupement isopropylamino, tertiobutylamino ou (1H-indolyl-3)-2 diméthyl-1,1 éthylamino et $R_1$ représente hydrogène ou méthyle.

**Revendications** pour les Etats contractants: AT et ES

1. Procédé de préparation de dérivés d'hydroxy-4 indole de formule générale:

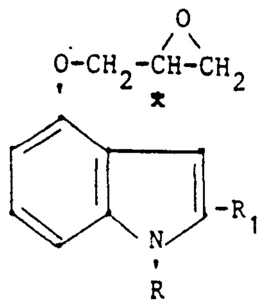

dans laquelle R représente un groupement protecteur labile et $R_1$ peut représenter:

- hydrogène,
- un radical alkyle, linéaire ou ramifié en $C_1$-$C_6$,
- un radical cycloalkyle en $C_3$-$C_6$,
- un radical alkoxy en $C_1$-$C_4$,
- un radical hydroxyalkyle en $C_1$-$C_4$,
- un radical (alkoxy en $C_1$-$C_4$) alkyle en $C_1$-$C_4$,
- un radical phényle éventuellement substitué par un atome d'halogène ou par un radical alkyle en $C_1$-$C_4$ ou alkoxy en $C_1$-$C_4$,
- un radical cyano,
- un radical de formule:

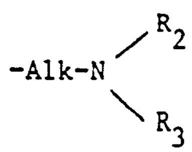 , 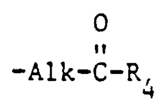 ou 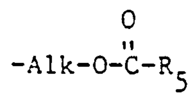

dans lequel: $R_2$ et $R_3$, identiques ou différents, représentent chacun hydrogène ou un radical alkyle en $C_1$-$C_4$,

$R_4$ représente hydroxy, un radical alkyle en $C_1$-$C_4$ ou alkoxy $C_1$-$C_4$ ou un radical 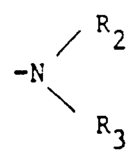

dans lequel $R_2$ et $R_3$ ont la même signification que précédemment,
$R_5$ représente un radical alkyle en $C_1$-$C_4$,
Alk représente une liaison simple ou un radical alkylène, linéaire ou ramifié, ayant de 1 à 4 atomes de carbone, caractérisé en ce que l'on fait réagir, dans un milieu approprié et à la température de reflux, un dérivé d'indole N-protege de formule generale:

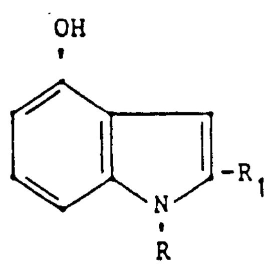

dans laquelle R et $R_1$ ont la même signification que ci-dessus avec l'épichlorhydrine ou l'épibromhydrine en presence d'un agent de métallation et éventuellement en présence d'un catalyseur de transfert de phase.

2. Procédé selon la revendication 1, dans lequel on prépare un composé de formule I dans lequel R représente un groupement alkylcarbonyle, arylcarbonyle, alkylsulfonyle ou arylsulfonyle.

3. Procédé selon la revendication 1, dans lequel on prépare un composé de formule I dans lequel R représente un radical formyle, acétyle, propionyle, benzoyle, méthanesulfonyle, benzenesulfonyle ou p-toluénesulfonyle.

4. Procédé selon la revendication 1, dans lequel on prépare un composé de formule I dans lequel $R_1$ représente hydrogène, un radical méthyle, éthyle, n-propyle, isopropyle, n-butyle, isobutyle, tertiobutyle, méthyl-1 propyle, n-pentyle, n-hexyle, cyclohexyle, méthoxy, éthoxy, hydroxyméthyle, hydroxy-1 éthyle, méthyloxyméthyle, phényle, chlorophényle, méthylphényle, méthoxyphényle, cyano, méthylamino, diméthylamino, méthylamino- ou éthylamino-éthyle, dimethylamino- ou diéthylamino-éthyle, carboxy, méthoxycarbonyle, éthoxycarbonyle, méthyl-2 méthoxycarbonyl-2 propyle, carbamoyle, N,N-diméthyl- ou N,N-diéthylcarbamoyle, acétyle, propionyle, acétoxyméthyle ou pivaloyloxyméthyle.

5. Procédé selon la revendication 1, dans lequel on prépare un composé de formule I dans lequel $R_1$ représente l'hydrogène ou méthyle et R représente benzénesulfonyle ou p-toluénesulfonyle.

6. Procédé selon la revendication 1, dans lequel on prépare le benzénesulfonyl-1 oxyranylméthoxy-4 indole.

7. Procédé selon la revendication 1, dans lequel on prépare le p-toluénesulfonyl-1 oxyranylméthoxy-4 indole.

8. Procédé de préparation de dérivés d'(amino-3 hydroxy-2 propoxy)-4 indole de formule générale:

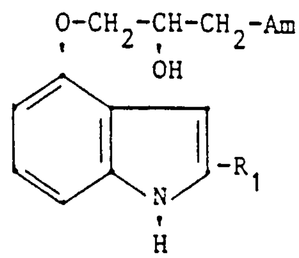

ainsi que de leurs sels, dans laquelle $R_1$ a la même signification que dans l'une des Revendications 1 à 5 et Am représente un groupement amino substitué ou non caractérisé en ce que l'on traite au reflux un dérivé d'hydroxy-4 indole tel que défini dans l'une des Revendications 1 à 5, avec une amine de formule générale:

H-Am

dans laquelle Am a la même signification que précédemment et ce, dans un alcool inférieur ou un excès d'amine ci-dessus, de manière à former un dérivé d'(hydroxy-2 propoxy)-4 indole de formule générale:

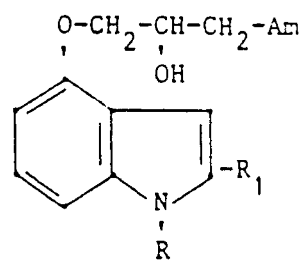

dans laquelle R et $R_1$ ont la même signification que dans les Revendications 1 à 5 et Am a la même signification que précédemment, dérivé que l'on déprotége par chauffage au reflux dans un alcool inférieur et en présence d'un hydroxyde de métal alcalin pour obtenir le dérivé désiré d'(amino-3 hydroxy-2 propoxy)-4 indole que l'on peut faire réagir, si on le désire, avec un acide pour former un sel de ce composé.

9. Procédé de préparation de dérivés d"(amino-3 hydroxy-2 propoxy)-4 indole de formule générale:

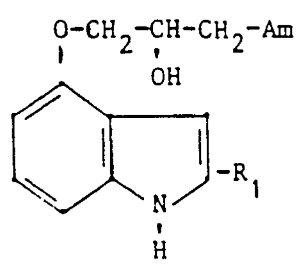

ainsi que de leurs sels, dans laquelle $R_1$ a la même signification que dans l'une des Revendications 1 à 5 et Am représente un groupement amino substitué ou non caractérisé en ce que:

- l'on fait réagir, dans un milieu approprié et à la température de reflux, un dérivé d'indole N-protégé de formule générale:

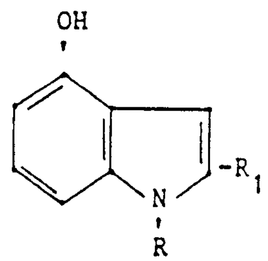

dans laquelle R et $R_1$ ont la même signification que dans les Revendications 1 à 5, avec l'épichlorhydrine ou l'épibromhydrine en présence d'un agent de métallation et d'un tris(dioxa-alkyl)amine comme catalyseur de transfert de phase pour obtenir un dérivé d'hydroxy-4 indole sous forme brute tel que défini à l'une des Revendications 1 à 7,
- l'on traite le dérivé d'hydroxy-4 indole brut ainsi obtenu avec une amine de formule générale:

H-Am

dans laquelle Am a la même signification que précédemment et ce, dans un alcool inférieur ou un excès d'amine ci-dessus, de manière à former un dérivé d'(hydroxy-2 propoxy)-4 indole de formule générale:

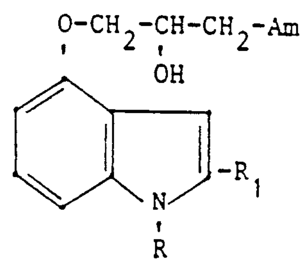

dans laquelle R et $R_1$ ont la même signification que dans les Revendications 1 à 5 et An a la même signification que précédemment,
- l'on déprotège le dérivé d'(hydroxy-2 propoxy)-4 indole ainsi obtenu, par chauffage au reflux dans un alcool inférieur et en présence d'un hydroxyde de métal alcalin pour obtenir le dérivé désiré d'(amino-3 hydroxy-2 propoxy)-4 indole que l'on peut faire réagir, si on le désire, avec un acide pour former un sel.

10. Procédé selon la Revendication 1 ou 9 caractérisé en ce que l'agent de métallation est un hydrure de métal alcalin, un hydroxyde de métal alcalin, un carbonate de métal alcalin ou un alcoolate de métal alcalin.

11. Procédé selon la Revendication 1 ou 9 caractérisé en ce que le catalyseur de transfert de phase est la tris(dioxa-3,6 heptyl)amine.

12. Procédé selon la Revendication 1 ou 9 caractérisé en ce que le milieu est monophasique ou diphasique.

13. Procédé selon la Revendication 1 ou 9 caractérisé en ce que Am représente un groupement isopropylamino, tertiobutylamino ou (1H-indolyl-3)-2 diméthyl-1,1 éthylamino et $R_1$ représente hydrogène ou méthyle.

**Patentansprüche** für die Vertragsstaaten: BE, CH, DE, FR, GB, GR, IT, LI, LU, NL, SE

1. 4-Hydroxyindol-Derivate der allgemeinen Formel I,

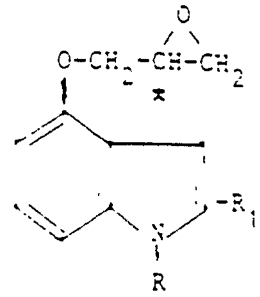

I

in der bedeuten:
R eine labile Schutzgruppe und
$R_1$ Wasserstoff, geradkettiges oder verzweigtes $C_{1-6}$-Alkyl, $C_{3-6}$-Cycloalkyl, $C_{1-4}$-Alkoxy, $C_{1-4}$-Hydroxyalkyl, ($C_{1-4}$-Alkoxy)-$C_{1-4}$-alkyl, ggf. mit einem Halogen oder einem $C_{1-4}$-Alkyl oder $C_{1-4}$-Alkoxy substituiertes Phenyl, Cyano oder einen Rest der Formeln:

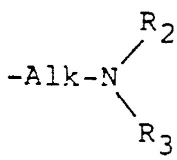 , 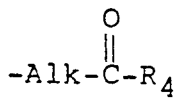 oder 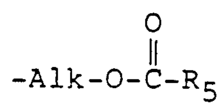

mit $R_2$ und $R_3$, gleich oder verschieden, Wasserstoff oder $C_{1-4}$-Alkyl,

$R_4$ Hydroxy, $C_{1-4}$-Alkyl oder $C_{1-4}$-Alkoxy oder einen Rest 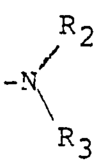 ,

wobei $R_2$ und $R_3$ die oben angegebene Bedeutung haben,
$R_5$ $C_{1-4}$-Alkyl und
Alk eine einfache Bindung oder geradkettiges oder verzweigtes $C_{1-4}$-Alkylen.

2. 4-Hydroxyindol-Derivate nach Anspruch 1 der Formel I, in der R Alkylcarbonyl, Arylcarbonyl, Alkylsulfonyl oder Arylsulfonyl ist.

3. 4-Hydroxyindol-Derivate nach Anspruch 1 der Formel I, in der R Formyl, Acetyl, Propionyl, Benzoyl, Methansulfonyl, Benzolsulfonyl oder p-Toluolsulfonyl ist.

4. 4-Hydroxyindol-Derivate nach Anspruch 1 der Formel I, in der $R_1$ Wasserstoff, Methyl, Ethyl, n-Propyl, Isopropyl, n-Butyl, Isobutyl, t-Butyl, 1-Methylpropyl, n-Pentyl, n-Hexyl, Cyclohexyl, Methoxy, Ethoxy, Hydroxymethyl, 1-Hydroxyethyl, Methyloxymethyl, Phenyl, Chlorphenyl, Methylphenyl, Methoxyphenyl, Cyano, Methylamino, Dimethylamino, Methylamino- oder Ethylaminoethyl, Dimethylamino- oder Diethylaminoethyl, Carboxy, Methoxycarbonyl, Ethoxycarbonyl, 2-Methyl-2-methoxycar-bonylpropyl, Carbamoyl, N,N-Dimethyl- oder N,N-Diethylcarbamoyl, Acetyl, Propionyl, Acetoxymethyl oder Pivaloyloxymethyl ist.

5. 4-Hydroxyindol-Derivate nach Anspruch 1 der Formel I, in der $R_1$ Wasserstoff oder Methyl und R Benzolsulfonyl oder p-Toluolsulfonyl ist.

6. 1-Benzolsulfonyl-4-oxyranylmethoxyindol.

7. 1-p-Toluolsulfonyl-4-oxyranylmethoxyindol.

8. Verfahren zur Herstellung der 4-Hydroxyindol-Derivate nach einem der Ansprüche 1 bis 7, gekennzeichnet durch:
Umsetzung in einem geeigneten Medium und bei Rückflußtemperatur eines N-geschützten Indolderivats der allgemeinen Formel IV,

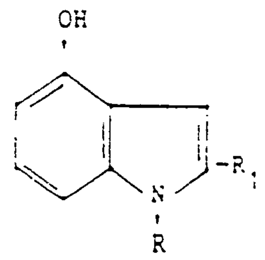

IV

in der R und $R_1$ die in den Ansprüchen 1 bis 5 angegebene Bedeutung haben,

mit Epichlorhydrin oder Epibromhydrin in Gegenwart eines Metallsalz bildenden Mittels und ggf. eines Phasentransferkatalysators.

9. Verfahren zur Herstellung von 4-(3-Amino-2-hydroxypropoxy)-indol-Derivaten der allgemeinen Formel III sowie ihrer Salze,

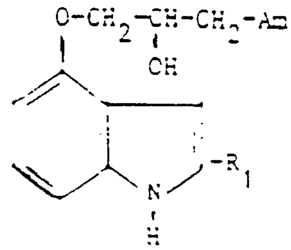

III

in der $R_1$ die in den Ansprüchen 1 bis 5 angegebene Bedeutung hat und Am eine ggf. substituierte Aminogruppe ist,

dadurch gekennzeichnet, daß

- ein 4-Hydroxyindol-Derivat nach einem der Ansprüche 1 bis 5 mit einem Amin der allgemeinen Formel II

H-Am II

in der Am die oben angegebene Bedeutung hat, in einem niedrigen Alkohol oder in einem Überschuß des Amins so unter Rückfluß erhitzt wird, daß ein 4-(2-Hydroxypropoxy)-indol-Derivat der allgemeinen Formel III gebildet wird,

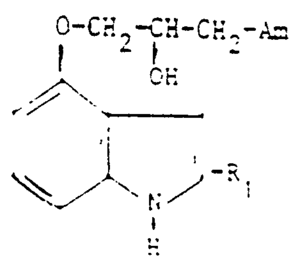

III

in der R und $R_1$ die in den Ansprüchen 1 bis 5 angegebene Bedeutung und Am die oben angegebene Bedeutung haben,
- die Schutzgruppe durch Erhitzen unter Rückfluß in einem niedrigen Alkohol und in Gegenwart eines Alkalimetallhydroxyds entfernt wird und
- das erhaltene gewünschte 4-(3-Amino-2-hydroxypropoxy)-indol-Derivat ggf. mit einer Säure zu einem Salz der Verbindung umgesetzt wird.

10. Verfahren zur Herstellung von 4-(3-Amino-2-hydroxy-propoxy)-indol-Derivat der allgemeinen Formel III sowie ihrer Salze,

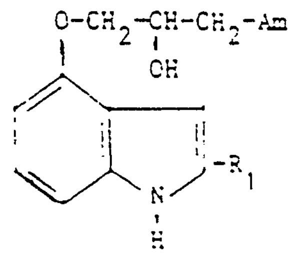

III

in der $R_1$ die in den Ansprüchen 1 bis 5 angegebene Bedeutung hat und Am eine ggf. substituierte Aminogruppe ist,
dadurch gekennzeichnet, daß

- ein N-geschütztes Indolderivat der allgemeinen Formel IV in einem geeigneten Medium und bei Rückflußtemperatur,

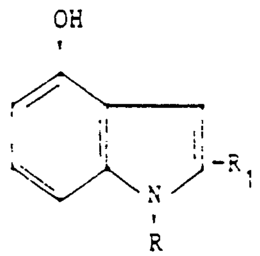

IV

in der R und $R_1$ die in den Ansprüchen 1 bis 5 angegebene Bedeutung haben, mit Epichlorhydrin oder Epibromhydrin in Gegenwart eines Metallsalz bildenden Mittels und von Tris(dioxa-alkyl)amin als Phasentransferkatalysator zu einem rohen 4-Hydroxyindol-Derivat nach einem der Ansprüche 1 bis 7 umgesetzt wird,
- das so erhaltene rohe 4-Hydroxyindol-Derivat mit einem Amin der allgemeinen Formel II,

H-Am II

in der Am die oben angegebene Bedeutung hat, in einem niedrigen Alkohol oder einem Überschuß des Amins so umgesetzt wird, daß ein 4-(2-Hydroxypropoxy)-indol-Derivat der allgemeinen Formel III gebildet wird,

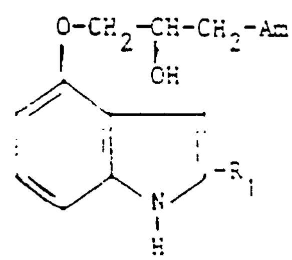

III

in der R und $R_1$ die in den Ansprüchen 1 bis 5 angegebene Bedeutung und Am die oben angegebene Bedeutung haben,
- die Schutzgruppe des so erhaltenen 4-(2-Hydroxypropoxy)-indol-Derivats durch Erhitzen unter Rückfluß in einem niedrigen Alkohol und in Gegenwart eines Alkalimetallhydroxyds entfernt wird und
- das gewünschte 4-(3-Amino-2-hydroxypropoxy)-indol-Derivat ggf. mit einer Säure zur Bildung eines Salzes umgesetzt wird.

11. Verfahren nach Anspruch 8 oder 10, dadurch gekennzeichnet, daß als Metallsalz bildendes Mittel ein Alkalimetallhydrid, Alkalimetallhydroxyd, Alkalimetallcarbonat oder Alkalimetallalkoholat verwendet wird.

12. Verfahren nach Anspruch 8 oder 10, dadurch gekennzeichnet, daß als Phasentransferkatalysator Tris(3,6-dioxaheptyl)amin verwendet wird.

13. Verfahren nach Anspruch 8 oder 10, dadurch gekennzeichnet, daß ein einphasiges oder zweiphasiges Medium verwendet wird.

14. Verfahren nach Anspruch 8 oder 10, dadurch gekennzeichnet, daß Verbindungen der Formel II oder III verwendet werden, in denen Am Isopropylamino, t-Butylamino oder 2-(3-1H-Indolyl)-1,1-dimethylethylamino und $R_1$ Wasserstoff oder Methyl ist.

**Patentansprüche** für die Vertragsstaaten: AT und ES

1. Verfahren zur Herstellung von 4-Hydroxyindol-Derivaten der allgemeinen Formel I

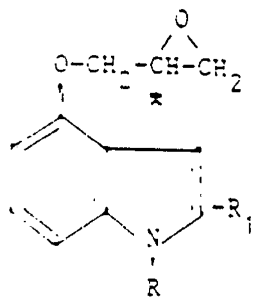

I

in der bedeuten:

R eine labile Schutzgruppe und

$R_1$ Wasserstoff, geradkettiges oder verzweigtes $C_{1-6}$-Alkyl, $C_{3-6}$-Cycloalkyl, $C_{1-4}$-Alkoxy, $C_{1-4}$-Hydroxyalkyl, ($C_{1-4}$-Alkoxy)-$C_{1-4}$-alkyl, ggf. mit einem Halogen oder einem $C_{1-4}$-Alkyl oder $C_{1-4}$-Alkoxy substituiertes Phenyl, Cyano oder einen Rest der Formeln:

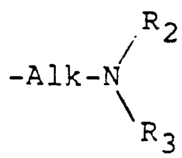 , 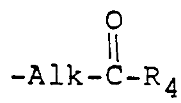 oder 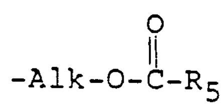

mit $R_2$ und $R_3$, gleich oder verschieden, Wasserstoff oder $C_{1-4}$-Alkyl,

$R_4$ Hydroxy, $C_{1-4}$-Alkyl oder $C_{1-4}$-Alkoxy oder einen Rest 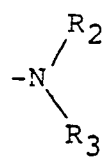 ,

wobei $R_2$ und $R_3$ die oben angegebene Bedeutung haben,

$R_5$ $C_{1-4}$-Alkyl und

Alk eine einfache Bindung oder geradkettiges oder verzweigtes $C_{1-4}$-Alkylen,

gekennzeichnet durch

Umsetzung in einem geeigneten Medium und bei Rückflußtemperatur eines N-geschützten Indolderivats der allgemeinen Formel IV

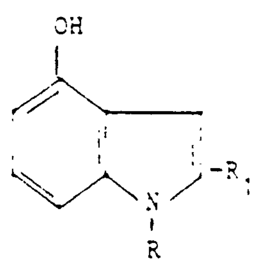

IV

in der R und $R_1$ die oben angegebene Bedeutung haben, mit Epichlorhydrin oder Epibromhydrin in Gegenwart eines Metallsalz bildenden Mittels und ggf. eines Phasentransferkatalysators.

2. Verfahren nach Anspruch 1, in dem eine Verbindung der Formel I hergestellt wird, in der R Alkylcarbonyl, Arylcarbonyl, Alkylsulfonyl oder Arylsulfonyl ist.

3. Verfahren nach Anspruch 1, in dem eine Verbindung der Formel I hergestellt wird, in der R Formyl, Acetyl, Propionyl, Benzoyl, Methansulfonyl, Benzolsulfonyl oder p-Toluolsulfonyl ist.

4. Verfahren nach Anspruch 1, in dem eine Verbindung der Formel I hergestellt wird, in der $R_1$ Wasserstoff, Methyl, Ethyl, n-Propyl, Isopropyl, n-Butyl, Isobutyl, t-Butyl, 1-Methylpropyl, n-Pentyl, n-Hexyl, Cyclohexyl, Methoxy, Ethoxy, Hydroxymethyl, 1-Hydroxyethyl, Methyloxymethyl, Phenyl, Chlorphenyl, Methylphenyl,

Methoxyphenyl, Cyano, Methylamino, Dimethylamino, Methylamino- oder Ethylaminoethyl, Dimethylamino- oder Diethylaminoethyl, Carboxy, Methoxycarbonyl, Ethoxycarbonyl, 2-Methyl-2-methoxycarbonylpropyl, Carbamoyl, N,N-Dimethyl- oder N,N-Diethylcarbamoyl, Acetyl, Propionyl, Acetoxymethyl oder Pivaloyloxymethyl ist.

5. Verfahren nach Anspruch 1, in dem eine Verbindung der Formel I hergestellt wird, in der $R_1$ Wasserstoff oder Methyl und R Benzolsulfonyl oder p-Toluolsulfonyl ist.

6. Verfahren nach Anspruch 1, in dem 1-Benzolsulfonyl-4-oxyranylmethoxyindol hergestellt wird.

7. Verfahren nach Anspruch 1, in dem 1-p-Toluolsulfonyl-4-oxyranylmethoxyindol hergestellt wird.

8. Verfahren zur Herstellung von 4-(3-Amino-2-hydroxypropoxy)-indol-Derivaten der allgemeinen Formel III sowie ihrer Salze,

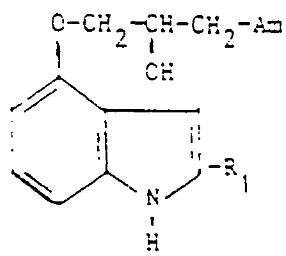

III

in der $R_1$ die in den Ansprüchen 1 bis 5 angegebene Bedeutung hat und Am eine ggf. substituierte Aminogruppe ist,

dadurch gekennzeichnet, daß

- ein 4-Hydroxyindol-Derivat, wie das eines der Ansprüche 1 bis 5, mit einem Aminder allgemeinen Formel II

H-Am II

in der Am die oben angegebene Bedeutung hat, in einem niedrigen Alkohol oder in einem Überschuß des Amins so unter Rückfluß erhitzt wird, daß ein 4-(2-Hydroxy-propoxy)-indol-Derivat der allgemeinen Formel III gebildet wird

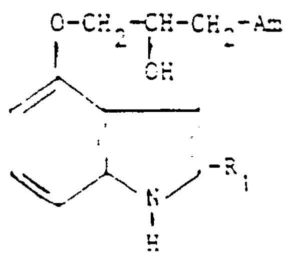

III

in der R und $R_1$ die in den Ansprüchen 1 bis 5 angegebene Bedeutung und Am die oben angegebene Bedeutung haben,
- die Schutzgruppe durch Erhitzen unter Rückfluß in einem niedrigen Alkohol und in Gegenwart eines Alkalimetallhydroxyds entfernt wird und
- das erhaltene gewünschte 4-(3-Amino-2-hydroxypropoxy)-indol-Derivat ggf. mit einer Säure zu einem Salz der Verbindung umgesetzt wird.

8. Verfahren zur Herstellung von 4-(3-Amino-2-hydroxy-propoxy)-indol-Derivat der allgemeinen Formel III sowie ihrer Salze,

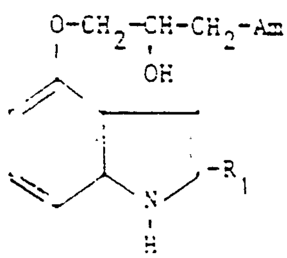

III

in der $R_1$ die in den Ansprüchen 1 bis 5 angegebene Bedeutung hat und Am eine ggf. substituierte Aminogruppe ist,

dadurch gekennzeichnet, daß

- ein N-geschütztes Indolderivat der allgemeinen Formel IV in einem geeigneten Medium und bei Rückflußtemperatur

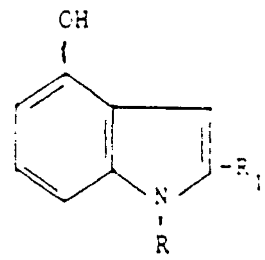

IV

in der R und $R_1$ die in den Ansprüchen 1 bis 5 angegebene Bedeutung haben, mit Epichlorhydrin oder Epibromhydrin in Gegenwart eines Metallsalz bildenden Mittels und von Tris(dioxa-alkyl)amin als Phasentransferkatalysator zu einem rohen 4-Hydroxyindol-Derivat nach einem der Ansprüche 1 bis 7 umgesetzt wird,

- das so erhaltene rohe 4-Hydroxyindol-Derivat mit einem Amin der allgemeinen Formel II

H-Am II

in der Am die oben angegebene Bedeutung hat, in einem niedrigen Alkohol oder einem Überschuß des Amins so umgesetzt wird, daß ein 4-(2-Hydroxypropoxy)-indol-Derivat der allgemeinen Formel III gebildet wird,

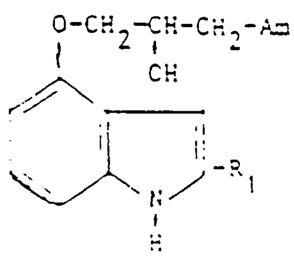

III

in der R und $R_1$ die in den Ansprüchen 1 bis 5 angegebene Bedeutung und Am die oben angegebene Bedeutung haben,

- die Schutzgruppe des so erhaltenen 4-(2-Hydroxypropoxy)-indol-Derivats durch Erhitzen unter Rückfluß in einem niedrigen Alkohol und in Gegenwart eines Alkalimetallhydroxyds entfernt wird und
- das gewünschte 4-(3-Amino-2-hydroxypropoxy)-indol-Derivat ggf. mit einer Säure zur Bildung eines Salzes umgesetzt wird.

10. Verfahren nach Anspruch 1 oder 9, dadurch gekennzeichnet, daß als Metallsalz bildendes Mittel ein Alkalimetallhydrid, Alkalimetallhydroxyd, Alkalimetallcarbonat oder Alkalimetallalkoholat verwendet wird.

11. Verfahren nach Anspruch 1 oder 9, dadurch gekennzeichnet, daß als Phasentransferkatalysator Tris(3,6-dioxaheptyl)amin verwendet wird.

12. Verfahren nach Anspruch 1 oder 9, dadurch gekennzeichnet, daß ein einphasiges oder zweiphasiges Medium verwendet wird.

13. Verfahren nach Anspruch 1 oder 8, dadurch gekennzeichnet, daß Verbindungen der Formel II oder III verwendet werden, in denen Am Isopropylamino, t-Butylamino oder 2-(3-1H-Indolyl)-1,1-dimethylethylamino und $R_1$ Wasserstoff oder Methyl ist.

**Claims** for the Contracting States: BE, CH, DE, FR, GB, GR, IT, LI, LU, NL, SE

1. 4-Hydroxyindole derivatives of general formula:

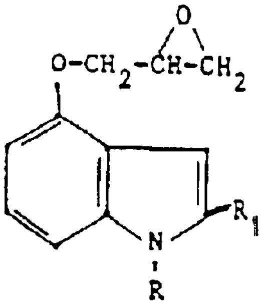

I

in which R represents a labile protective group and $R_1$ can represent:

- hydrogen
- a straight- or branched-chain $C_{1-6}$ alkyl radical,
- a $C_{3-6}$ cycloalkyl radical,
- a $C_{1-4}$ alkoxy radical,
- a $C_{1-4}$ hydroxyalkyl radical
- a $C_{1-4}$ alkoxy $C_{1-4}$ alkyl radical,
- a phenyl radical optionally substituted with a halogen atom or with a $C_{1-4}$ alkyl or $C_{1-4}$ alkoxy radical,
- a cyano radical,
- a radical of formula: 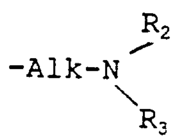 , 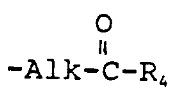 or

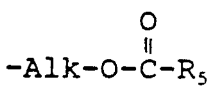 in which:

in which:

$R_2$ and $R_3$, which may be identical or different, each represent hydrogen or a $C_{1-4}$ alkyl radical,

$R_4$ represents a hydroxy group, a $C_{1-4}$ alkyl or $C_{1-4}$ alkoxy radical or a radical 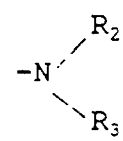

in which $R_2$ and $R_3$ have the same meaning as above,

$R_5$ represents a $C_{1-4}$ alkyl radical, and

Alk represents a single bond or a straight- or branched-chain alkylene radical having from 1 to 4 carbon atoms.

2. 4-Hydroxyindole derivatives according to claim 1, in which R represents an alkylcarbonyl, arylcarbonyl, alkylsulphonyl or arylsulphonyl group.

3. 4-Hydroxyindole derivatives according to claim 1, in which R represents a formyl, acetyl, propionyl, benzoyl, methanesulphonyl, benzenesulphonyl or p-toluenesulphonyl radical.

4. 4-Hydroxyindole derivatives according to claim 1, in which $R_1$ represents hydrogen or a methyl, ethyl, n-propyl, isopropyl, n-butyl, isobutyl, tert.-butyl, 1-methylpropyl, n-pentyl, n-hexyl, cyclohexyl, methoxy, ethoxy, hydroxymethyl, 1-hydroxyethyl, methyloxymethyl, phenyl, chlorophenyl, methylphenyl, methoxyphenyl, cyano, methylamino, dimethylamino, methylamino, or ethylaminoethyl, dimethylamino- or diethylaminoethyl, carboxy, methoxycarbonyl, ethoxycarbonyl, (2-methyl-2-methoxycarbonyl)-propyl, carbamoyl, N,N-dimethyl- or N,N-diethylcarbamoyl, acetyl, propionyl, acetoxymethyl or pivaloyloxymethyl radical.

5. 4-Hydroxyindole derivatives according to claim 1, in which $R_1$ represents hydrogen or a methyl radical and R represents a benzenesulphonyl or p-toluenesulphonyl radical.

6. 1-Benzenesulphonyl-4-oxyranylmethoxy-indole.

7. 1-(p-Toluenesulphonyl)-4-oxyranylmethoxy-indole.

8. Process for preparing 4-hydroxindole derivatives according to any one of claims 1 to 7, whereby an N-protected indole derivative of general formula:

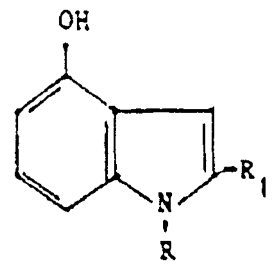

in which R and $R_1$ have the same meaning as in claims 1 to 5, is reacted, in a suitable medium and at the reflux temperature, with epichlorohydrin or epibromohydrin in the presence of a metallating agent and optionally in the presence of a phase transfer catalyst.

9. Process for preparing 4-(3-amino-2-hydroxypropoxy)-indole derivatives of general formula:

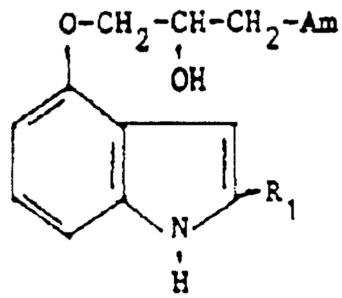

in which $R_1$ has the same meaning as in any one of claims 1 to 5 and Am represents a substituted or unsubstituted amino group, and their salts, whereby a 4-hydroxyindole derivative according to any one of claims 1 to 5 is treated under reflux with an amine of general formula:

H-Am

in which Am has the same meaning as above, this being carried out in a lower alcohol or an excess of the above amine, so as to form a 4-(2-hydroxypropoxy)indole derivative of general formula:

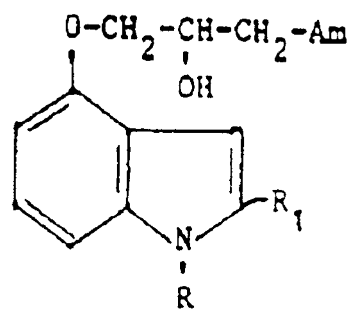

in which R and $R_1$ have the same meaning as in claims 1 to 5 and Am has the same meaning as above, said derivative is then deprotected by heating under reflux in a lower alcohol and in the presence of an alkali metal hydroxide to obtain the desired 4-(3-amino-2-hydroxypropoxy)indole derivative which, if desired, is reacted with an acid to form a salt thereof.

10. Process for preparing 4-(3-amino-2-hydroxypropoxy)indole of general formula:

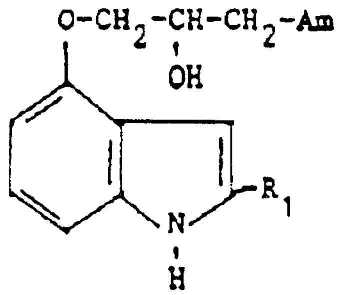

in which $R_1$ has the same meaning as in any one of claims 1 to 5 and Am represents a substituted or unsubstituted amino group, and their salts, whereby:

- a N-protected indole derivative of general formula:

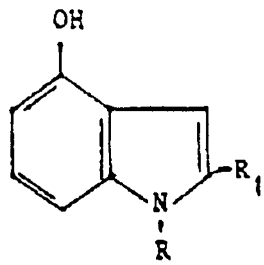

in which R and $R_1$ have the same meaning as in any one of claims 1 to 5, is reacted at reflux temperature and in an appropriate medium, with epichlorohydrin or epibromohydrin in the presence of a metallating agent and a tris(dioxa-alkyl)amine as a phase transfer catalyst to obtain a 4-hydroxyindole derivative, in crude form, according to any one of claims 1 to 7,
- the crude 4-hydroxyindole derivative thus obtained is treated with an amine of general formula:

H-Am

in which Am has the same meaning as above, this reaction being carried out in a lower alcohol or an excess of the above amine, so as to form a 4-(2-hydroxypropoxy)indole derivative of general formula:

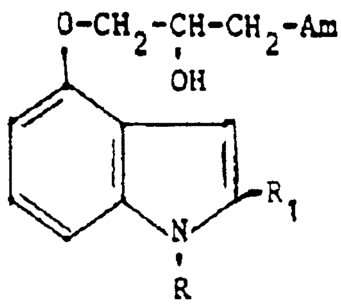

in which R and $R_1$ have the same meaning as in Claims 1 to 5 and Am has the same meaning as above,
- the 4-(2-hydroxypropoxy)indole derivative thus obtained is deprotected by heating under reflux in a lower alcohol and in the presence of an alkali metal hydroxide to obtain the desired 4-(3-amino-2-hydroxypropoxy)indole derivative which, if desired, may be reacted with an acid to form a salt thereof.

11. Process according to claim 8 or 10 whereby the metallating agent is an alkali metal hydride, an alkali metal hydroxide, an alkali metal carbonate or an alkali metal alcoholate.

12. Process according to claim 8 or 10 whereby the phase transfer catalyst is tris(3,6-dioxaheptyl)amine.

13. Process according to claim 8 or 10 whereby the medium is a single-phase or two-phase medium.

14. Process according to claim 8 or 10 whereby Am represents an isopropylamino, tert.-butylamino or 2-(1H-indol-3-yl)-1,1-dimethylethylamino group and $R_1$ represents hydrogen or a methyl radical.

**Claims** for the Contracting States: AT and ES

1. Process for preparing 4-hydroxyindole derivatives of general formula:

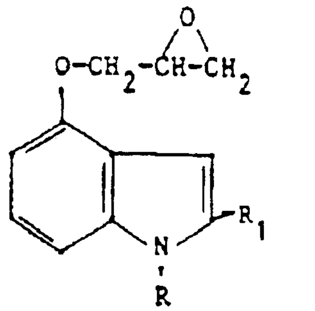

I

in which R represents a labile protective group and $R_1$ can represent:

- hydrogen
- a straight- or branched-chain $C_{1-6}$ alkyl radical,
- a $C_{3-6}$ cycloalkyl radical,
- a $C_{1-4}$ alkoxy radical,
- a $C_{1-4}$ hydroxyalkyl radical
- a $C_{1-4}$ alkoxy $C_{1-4}$ alkyl radical,
- a phenyl radical optionally substituted with a halogen atom or with a $C_{1-4}$ alkyl or $C_{1-4}$ alkoxy radical,
- a cyano radical,
- a radical of formula 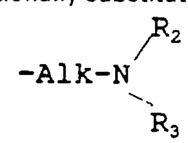 , 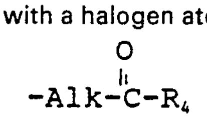 or

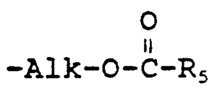

in which:

$R_2$ and $R_3$, which may be identical or different, each represent hydrogen or a $C_{1-4}$ alkyl radical,

$R_4$ represents a hydroxy group, a $C_{1-4}$ alkyl or $C_{1-4}$ alkoxy radical or a radical 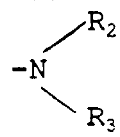

in which $R_2$ and $R_3$ have the same meaning as above,

$R_5$ represents a $C_{1-4}$ alkyl radical, and

Alk represents a single bond or a straight- or branched-chain alkylene radical having from 1 to 4 carbon atoms, whereby an N-protected indole derivative of general formula:

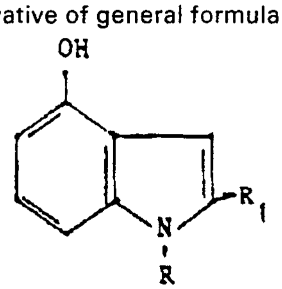

in which R and $R_1$ have the same meaning as above, is reacted, in a suitable medium and at the reflux temperature, with epichlorohydrin or epibromohydrin in the presence of a metallating agent and optionally in the presence of a phase transfer catalyst.

2. Process according to claim 1, whereby a compound of formula I is prepared, in which R represents an alkylcarbonyl, arylcarbonyl, alkylsulphonyl or arylsulphonyl group.

3. Process according to claim 1, whereby a compound of formula I is prepared, in which R represents a formyl, acetyl, propionyl, benzoyl, methanesulphonyl, benzenesulphonyl or p-toluenesulphonyl radical.

4. Process according to claim 1, whereby a compound of formula I is prepared, in which $R_1$ represents hydrogen or a methyl, ethyl, n-propyl, isopropyl, n-butyl, isobutyl, tert.butyl, 1-methylpropyl, n-pentyl, n-hexyl, cyclohexyl, methoxy, ethoxy, hydroxymethyl, 1-hydroxyethyl, methyloxymethyl, phenyl, chlorophenyl, methylphenyl, methoxyphenyl, cyano, methylamino, dimethylamino, methylamino- or ethylaminoethyl, dimethylamino or diethylaminoethyl, carboxy, methoxycarbonyl, ethoxycarbonyl, (2-methyl-2-methoxycarbonyl)-propyl, carbamoyl, N,N-dimethyl- or N,N-diethylcarbamoyl, acetyl, propionyl, acetoxymethyl or pivaloyloxymethyl radical.

5. Process according to claim 1, whereby a compound of formula I is prepared, in which $R_1$ represents hydrogen or methyl and R represents benzenesulphonyl or p-toluenesulphonyl.

6. Process according to claim 1, in which 1-benzenesulphonyl-4-oxyranylmethoxy-indole is prepared.

7. Process according to claim 1, in which 1-(p-toluenesulphonyl)-4-oxyranylmethoxy-indole is prepared.

8. Process for preparing 4-(3-amino-2-hydroxypropoxy)-indole derivatives of general formula:

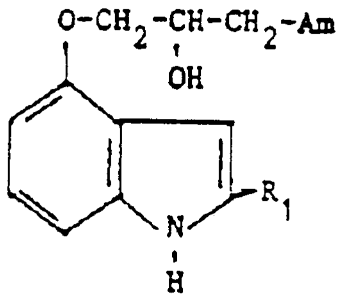

in which $R_1$ has the same meaning as in any one of claims 1 to 5 and Am represents a substituted or unsubstituted amino group, and their salts, whereby a 4-hydroxyindole derivative of the kind as in any one of claims 1 to 5 is treated under reflux, with an amine of general formula:

H-Am

in which Am has the same meaning as above, this being carried out in a lower alcohol or an excess of the above amine, so as to form a 4-(2-hydroxypropoxy)indole derivative of general formula:

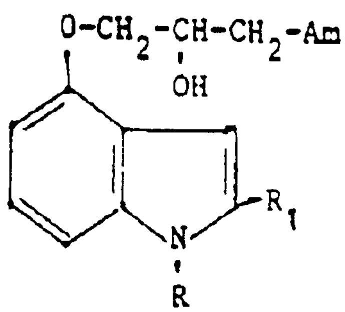

in which R and $R_1$ have the same meaning as in claims 1 to 5 and Am has the same meaning as above, said derivative is then deprotected by heating under reflux in a lower alcohol and in the presence of an alkali metal hydroxide to obtain the desired 4-(3-amino-2-hydroxypropoxy)indole derivative which, if desired, is reacted with an acid to form a salt thereof.

9. Process for preparing 4-(3-amino-2-hydroxy-propoxy)indole of general formula:

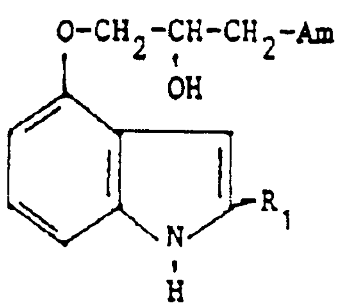

in which $R_1$ has the same meaning as in any one of claims 1 to 5 and Am represents a substituted or unsubstituted amino group, and their salts, whereby:

- a N-protected indole derivative of general formula:

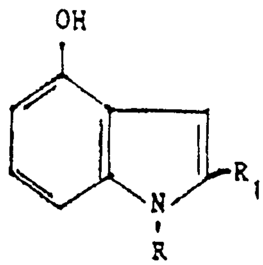

in which R and $R_1$ have the same meaning as in any one of claims 1 to 5, is reacted at reflux temperature and in an appropriate medium, with epichlorohydrin or epibromohydrin in the presence of a metallating agent and a tris(dioxa-alkyl)amine as a phase transfer catalyst to obtain a 4-hydroxyindole derivative, in crude form, of the kind as in any one of claims 1 to 7,

- the crude 4-hydroxyindole derivative thus obtained is treated with an amine of general formula:

H-Am

in which Am has the same meaning as above, this being performed in a lower alcohol or an excess of the above amine, so as to form a 4-(2-hydroxypropoxy)indole derivative of general formula:

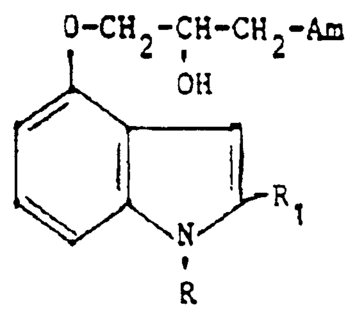

in which R and $R_1$ have the same meaning as in Claims 1 to 5 and Am has the same meaning as above,

- the 4-(2-hydroxypropoxy)indole derivative thus obtained is deprotected by heating under reflux in a lower alcohol and in the presence of an alkali metal hydroxide to obtain the desired 4-(3-amino-2-hydroxy-propoxy)indole derivative which, if desired, may be reacted with an acid to form a salt thereof.

10. Process according to claim 1 or 9 whereby the metallating agent is an alkali metal hydride, an alkali metal hydroxide, an alkali metal carbonate or an alkali metal alcoholate.

11. Process according to claim 1 or 9 whereby the phase transfer catalyst is tris(3,6-dioxaheptyl)amine.

12. Process according to claim 1 or 9 whereby the medium is a single-phase or two-phase medium.

13. Process according to claim 1 or 9 whereby Am represents an isopropylamino, tert.-butylamino or 2-(1H-indol-3-yl)-1,1-dimethylethylamino group and $R_1$ represents hydrogen or a methyl radical.